# EUROPÄISCHE PATENTSCHRIFT

(11) **EP 2 919 791 B1**
(45) Veröffentlichungstag und Bekanntmachung des Hinweises auf die Patenterteilung: **22.03.2017**
(21) Anmeldenummer: 13786226.4
(22) Anmeldetag: 01.11.2013
(51) Int. Cl.: A61K 31/353, A61K 31/724, A61K 31/737, A61K 9/08, A61K 47/00, A61K 45/06, A61P 29/00, A61P 37/06

(54) **DELPHINIDINKOMPLEX ALS ANTIPHLOGISTISCHER ODER IMMUNSUPPRESSIVER WIRKSTOFF**
DELPHINIDINCOMPLEX AS ANTIPHLOGISTIC OR IMMUNOSUPPRESSIVE AGENT
COMPLEXE DE DELPHINIDIN COMME PRINCIPE ACTIF ANTIPHLOGISTIQUE OU IMMUNOSUPPRESSIVE

(30) Priorität: 15.11.2012 EP 12192717
(43) Veröffentlichungstag der Anmeldung: 23.09.2015
(73) Patentinhaber: SapioTec GmbH, 97082 Würzburg (DE)
(72) Erfinder: ROEWER, Norbert, 97082 Würzburg (DE); BROSCHEIT, Jens, 97209 Würzburg (DE)
(74) Vertreter: Glawe, Delfs, Moll
(86) Internationale Anmeldenummer: PCT/EP2013/072856
(87) Internationale Veröffentlichungsnummer: WO 2014/075935

(56) Entgegenhaltungen:
- WO-A1-2008/126979
- WO-A1-2014/060548
- WO-A2-2011/048479
- JP-A- 2009 167 174
- KR-A- 20070 026 284
- KR-A- 20070 028 640
- US-A1- 2009 270 348
- BERTUGLIA S ET AL: "Microvascular effects of a natural flavonoid (IdB 1056) during ischemia-reperfusion injury", PHARMACOLOGICAL RESEARCH, ACADEMIC PRESS, LONDON, GB, Bd. 22, 1. September 1990 (1990-09-01), Seite 52, XP025956653, ISSN: 1043-6618, DOI: 10.1016/S1043-6618(09)80104-7 [gefunden am 1990-09-01]
- SCARABELLI T M ET AL: "Targeting STAT1 by myricetin and delphinidin provides efficient protection of the heart from ischemia/reperfusion-induced injury", FEBS LETTERS, ELSEVIER, AMSTERDAM, NL, Bd. 583, Nr. 3, 4. Februar 2009 (2009-02-04), Seiten 531-541, XP025923237, ISSN: 0014-5793, DOI: 10.1016/J.FEBSLET.2008.12.037 [gefunden am 2008-12-29]
- UEDA H ET AL: "EVALUATION OF A SULFOBUTYL ETHER BETA-CYCLODEXTRIN AS A SOLUBILIZING/STABILIZING AGENT FOR SEVERAL DRUGS", DRUG DEVELOPMENT AND INDUSTRIAL PHARMACY, NEW YORK, NY, US, Bd. 24, Nr. 9, 1. September 1998 (1998-09-01), Seiten 863-867, XP009040590, ISSN: 0363-9045, DOI: 10.3109/03639049809088532 in der Anmeldung erwähnt

## Beschreibung

Die Erfindung betrifft die Verwendung von Zusammensetzungen umfassend einen Komplex aus Delphinidin und einem Sulfoalkylether-β-Cyclodextrin als Antiphlogistikum und/oder Immunsuppressivum.

Zusammensetzungen enthaltend Delphinidin-Komplexe mit Sulfoalkylether-**β**-Cyclodextrin werden in der WO2014/060548 (Stand der Technik gemäß Art. 54(3) EPÜ) offenbart.

Reaktionen des Immunsystems werden durch die Komponenten des angeborenen und erworbenen Immunsystems vermittelt. Das angeboren unspezifische Immunsystem umfasst alle Reaktionen auf antigene und körperfremde Stimuli (Bakterien, Viren, Pilze, etc.), die von Makrophagen, Monocyten und Granulozyten ausgehen, sowie humorale Abwehrmechanismen einschließlich Interferon, Defensine und Akute-Phase-Proteine. Das erworbene oder adaptive Immunsystem umfasst alle spezifischen Abwehrreaktionen, die von Lymphozyten, insbesondere von B-Lymphozyten (B-Zellen) und T-Lymphozyten (T-Zellen), ausgehen. Unter bestimmten Bedingungen können die Reaktionen des Immunsystems selbst Ursache von Erkrankungen oder krankheitsrelevanten Zuständen sein. Solche Erkrankungen oder Zustände sind z.B. akute und chronische Entzündungen, Sepsis, Autoimmunerkrankungen oder Abstoßungsreaktionen nach Organ-, Zell- oder Gewebetransplantation. Bei diesen und anderen Zuständen, in denen eine inadäquate oder unerwünschte Immunantwort auftritt, ist aus klinischer Sicht eine Immunsuppression mittels Antiphlogistika bzw. Immunsuppressiva erforderlich.

Eine antiphlogistisch oder antiinflammatorisch wirkende Substanz ist eine Substanz, die in der Lage ist, partiell oder total, unmittelbar oder zeitversetzt eine Entzündung oder eine ihrer Manifestationen zu vermindern oder zu hemmen. Eine antiinflammatorisch wirkende Substanz kann entweder auf lösliche Mediatoren wie Zytokine gerichtet sein oder die Expression von bestimmten an einer Entzündungsreaktion beteiligten zellulären Oberflächenrezeptoren wie z.B. MHC-Klasse II Moleküle modulieren. Eine immunsuppressorisch wirkende Substanz ist eine Substanz, deren Wirkung auf das Immunsystem zu einer unmittelbaren oder verzögerten Reduzierung der Aktivität des immunologischen Systems führt. Eine immunsuppressorisch wirkende Substanz wird dann eingesetzt, wenn es zu einer überschießenden Immunantwort kommt z.B. gegen körpereigene Moleküle oder Gewebe oder gegen transplantiertes Gewebe, z.B. Autoimmunerkrankungen, Typ I Diabetes, Multiple Sklerose oder Rheumatoide Arthritis. Derzeit verwendete Antiphlogistika sind z.B. Azetylsalizylsäure, Diclophenac, Indometacin oder Glukokortikoide. Aus der Gruppe der Immunsuppressiva werden Zellteilungshemmer (Azathioprin) Calcineurin-Inhibitoren (Tacrolimus, Ciclosporin, Pimecrolimus) und Hydrocortison eingesetzt.

Im klinischen Alltag wird Hydrocortison zur Entzündungshemmung und Immunsuppression eingesetzt und beispielsweise in den Enddarm zur Linderung entzündlicher Symptome bei chronisch-entzündlichen Darmerkrankungen, wie Colitis ulcerosa, Morbus Crohn oder bei anderen Entzündungen des unteren Darmabschnitts wie der Proktosigmoiditis örtlich angewendet. Ebenfalls bekannt ist die Verwendung von Hydrocortison in Cremes und Salben zur Auftragung auf die Haut bei entzündlichen oder allergischen Hauterkrankungen wie Ekzemen, Neurodermitis, Schuppenflechte, Sonnenbrand, Hautinfektionen und Insektenstichen, um eine Linderung von Beschwerden wie Juckreiz oder Entzündungen zu bewirken.

Während die kurzzeitige Gabe auch hoher Hydrocortisongaben vom Körper in der Regel noch gut toleriert wird, treten bei längerer Behandlung und insbesondere bei innerlicher Gabe des Wirkstoffs unerwünschte Nebenwirkungen auf. Die innerliche Gabe des Wirkstoffes hat zwei wesentliche Nebenwirkungen. So fördert Hydrocortison zum einen die Wasserrückaufnahme aus dem Urin ins Blut. Dadurch vergrößert sich die Blutmenge, der Druck in den Blutgefäßen erhöht sich und damit der Blutdruck. Auf diese Weise kann es auch dauerhaft zu Bluthochdruck kommen. Zum anderen kann Hydrocortison Herzrhythmusstörungen auslösen. Hydrocortison steigert die Kaliumausscheidung mit dem Urin. So kann ein Kalium-Mangel entstehen, der Herzrhythmusstörungen begünstigt. Deshalb werden solche Präparate in der klinischen Praxis üblicherweise nur ab dem sechsten Lebensjahr und maximal zwei Wochen lang angewendet.

Aufgabe der vorliegenden Erfindung ist es, ein wirksames Antiphlogistikum und/oder Immunsuppressivum als Alternative oder Ergänzung zu aus dem Stand der Technik bekannten Antiphlogistika oder Immunsuppressiva wie Hydrocortison zur Verfügung zu stellen.

Gelöst wird diese Aufgabe durch die in den unabhängigen Ansprüchen beanspruchten Zusammensetzungen und Verwendungen, wobei vorteilhafte Ausführungsformen der Erfindung in den Unteransprüchen offenbart sind. Dass diese Aufgabe in der Tat gelöst wird, belegen die *in-vitro* und *in-vivo* Versuchsergebnisse zu der antiphlogistischen und antiinflammatorischen Wirkung von Delphinidin und Delphinidin-Sulfoethylether-β-Cyclodextrin in den Beispielen 6-11.

Zunächst seien einige im Rahmen der Erfindung verwendete Begriffe erläutert.

Der erfindungsgemäße Komplex oder die erfindungsgemäße Zusammensetzung werden zur Behandlung eines Objekts oder Individuums eingesetzt, das an einer Indikation leidet und/oder einer prophylaktischen Behandlung bedarf, die vorzugsweise ausgewählt ist aus der Gruppe bestehend aus
- entzündlichen Zuständen oder entzündlichen Erkrankungen,
- mit entzündlichen Gewebeveränderungen assozierte Erkrankungen,
- Abstoßungsreaktionen nach einer Transplantation und
- Autoimmunerkrankungen.

"Entzündliche Zustände oder entzündliche Erkrankungen" im Sinne der vorliegenden Erfindung umfassen Indikation wie beispielsweise rheumatoide Arthritis, chronische Polyarthritis, juvenile idiopathische Arthritis, Spondylitis, Osteoarthritis, Sepsis, Septischer Schock, Zerebrale Malaria, eine chronisch entzündliche Lungenerkrankung, Silikose, Sarkoidose, Reperfusionssyndrom, neurodegenerative oder neuroinflammatorische Erkrankungen, wie Crohn'sche Erkrankung, Multiple Sklerose und Parkinson, Colitis ulzerosa, Fieber bei Infektionen und auch Depressionen, die ebenfalls durch entzündliche Gewebereaktionen verursacht werden [Pace et al. (2006), Inreased stress-induced inflammatory responses in male patients with major depression and increased early life stress. Am. J. Psychiatry. 163(9):1630-3].

Inflammatorische Reaktionen sind ebenfalls im Zuge einer myokardialen und/oder zerebralen Minderdurchblutung (Ischämie), insbesondere nach wiederhergestellter Durchblutung (Reperfusion), zu beobachten, bei der die nekrotische Schwellung der Zellen zum Aufbrechen der Zellmembran und damit einhergehender Freisetzung der Bestandteile des Zytoplasmas kommt, welche eine inflammatorische Reaktion verursachen. Dem erfindungsgemäßen Komplex oder der erfindungsgemäßen Zusammensetzung kommt damit erfindungsgemäß auch eine Verwendung zu, die als Gewebe- und/oder Organprotektion im Kontext von zerebraler und/oder myokardialer Ischämie angesehen werden kann und mit den klinischen Krankheitsbildern des Schlaganfalls und des Myokoardinfarkts korrelieren. Gemäß einer bevorzugten Ausführungsform der Erfindung wird durch die Verwendung des erfindungemäßen Komplexes oder der erfindungemäßen Zusammensetzung von dem betroffenen Gewebe oder Organ zusätzlicher Schaden (Reperfusionsparadox) abgewendet bzw. gemildert, wie in den Untersuchungen des Ausführungsbeispiels 10 belegt.

Von den Atherosklerose assoziierte Erkrankungen, unter die insbesondere Schlaganfall, koronare Herzkrankheit (KHK) und periphere arterielle Verschlusskrankheit subsumiert werden, sind Menschen in den Industrieländern im erheblichen Maße betroffen. Derzeit sterben jährlich mehr als 7 Millionen Menschen an den Folgen ischämischer Herzkrankheiten. KHK verursacht eine mangelhafte Durchblutung und damit einhergehende nicht ausreichende Versorgung des Herzmuskels mit Sauerstoff (myokardiale Ischämie), mit der Folge, dass es zu Myokardinfarkt und Herztod kommen kann. Die KHK wird in 6 Verlaufsformen unterteilt: die stabile Angina pectoris, das akute Koronarsyndrom (instabile Angina pectoris/Herzenge und akuter Myokardinfarkt/Herzinfarkt), der plötzliche Herztod, die chronische Herzinsuffizienz, Herzrhythmusstörungen sowie die stumme Myokardischämie. Mit fortschreitender myokardialer Ischämie folgt der Zelluntergang der Kardiozyten durch "zufälligen" (Nekrose) und "programmierten" (Apoptose) Zelltod, wobei die nekrotische Schwellung der Zellen zum Aufbrechen der Zellmembranen unter Freisetzung der für die inflammatorische Reaktion verantwortlichen Zytoplasmabestandteile führt.

Unter "mit entzündlichen Gewebeveränderungen assoziierte Erkrankungen" fallen Indikationen wie aus der Gruppe bestehend aus Alzheimer, Parkinson und Krebs.

Der Begriff "Objekt" umfasst lebende Tiere und Menschen.

Der Begriff "Zusammensetzung umfassend einen Komplex aus Delphinidin und einem Sulfoalkylether-β-Cyclodextrin und/oder Delphinidin oder dessen Salze" schließt die Zusammensetzung als Monopräparat, d.h. ohne weitere therapeutisch aktive Komponenten ein. Alternativ kann die Zusammensetzung mindestens eine weitere therapeutisch aktive Substanz umfassen. Diese weitere therapeutisch wirksame Substanz ist vorzugsweise ausgewählt aus der Gruppe der Antiphlogistika, der Antikörper gegen inflammatorische Zytokine, der löslichen Rezeptoren inflammatorischer Zytokine oder der Immunosuppressiva und besonders bevorzugt ausgewählt aus der Gruppe bestehend aus Acetylsalicylsäure, Diclophenac, Indometacin, Ciclosporin, Azathioprin, Bortezomib, Melphalan, Prednison, Vincristin, Carmustin, Cyclophosphamid, Dexamethason, Thalidomid, Doxorubicin, Cisplatin, Etoposid und Cytarabin.

Die vorliegende Erfindung bezieht sich auch auf Verfahren zur Behandlung eines Objekts, das an einem entzündlichen Zustand oder einer entzündlichen Erkrankung, an einer mit entzündlichen Gewebeveränderungen assozierten Erkrankung, einer Abstoßungsreaktion nach einer Transplantation oder einer Autoimmunerkrankung leidet, wobei dem Objekt eine therapeutisch wirksame Menge der erfindungsgemäßen Zusammensetzung verabreicht wird. Wie bereits erwähnt, kann die erfindungsgemäße Zusammensetzung allein oder in Kombination mit mindestens einem anderen therapeutischen Mittel verabreicht werden. Die erfindungsgemäße Zusammensetzung kann gleichzeitig mit dem anderen therapeutischen Mittel, welches Bestandteil derselben Zusammensetzung sein kann oder in einer anderen Zusammensetzung bereitgestellt wird, verabreicht werden. Alternativ kann die erfindungsgemäße Zusammensetzung vor oder nach der Verabreichung des anderen therapeutischen Mittels verabreicht werden. Die erfindungsgemäße Zusammensetzung kann über den gleichen oder einen anderen Verabreichungsweg wie das andere therapeutische Mittel verabreicht werden.

Der Begriff "Behandlung" bedeutet im Sinne der vorliegenden Erfindung das ganze oder teilweise Erreichen der nachfolgend genannten Ergebnisse: Ganze oder teilweise Reduktion des Krankheitsbildes; Verbesserung mindestens eines der klinischen Symptome oder mit der Krankheit assoziierten Indikatoren; Verzögerung, Unterdrückung oder Schutz vor dem Fortschreiten der Krankheit; oder ganze oder teilweise Verzögerung, Unterdrückung oder Schutz vor dem Ausbrechen oder Entstehung der Krankheit. Das zu behandelnde Objekt ist ein Mensch oder Tier, vorzugsweise ein Säugetier. Die veterinärmedizinische Behandlung umfasst neben der Behandlung von Nutz- oder Wildtieren (z.B. Schafe, Katzen, Pferde, Kühe, Schweine) auch Labortiere (z.B. Ratten, Mäuse, Meerschweine, Affen).

Die erfindungsgemäße Zusammensetzung wird vorzugsweise als pharmazeutische Zusammensetzung bereitgestellt und verabreicht. Der Begriff "pharmazeutische Zusammensetzung" umfasst ein oder mehrere Wirkstoffe und ein oder mehrere inerte Inhaltstoffe, die als Träger für den Wirkstoff bzw. die Wirkstoffe fungieren. Die pharmazeutischen Zusammensetzungen erlauben es, den erfindungsgemäßen Komplex oder die erfindungsgemäße Zusammensetzung oral, rektal, parenteral, einschließlich intraperitoneal, perkutan, subkutan, intramuskulär, intravenös, ophthalmisch, pulmonal oder nasal zu verabreichen. Eine parenterale Verabreichungsform kann beispielsweise eine Tablette, Kapsel, Lösung, Suspension oder Dispersion sein. Eine ophthalmische, pulmonale oder nasale Verabreichungsform kann beispielsweise ein Aerosol, Lösung, Creme, Paste, Lotion, Gel, Salbe, Suspension oder Dispersion sein. Entsprechende Techniken für die Formulierung und Verabreichung sind aus dem Stand der Technik bekannt, siehe beispielsweise "Remington's Pharmaceutical Sciences" (Mack Publishing Co, Easton Pa.). Zum Beispiel können die erfindungsgemäßen Zusammensetzungen einem Subjekt intravenös mittels eines pharmazeutisch akzeptablen Trägers (z.B. physiologische Salzlösung) verabreicht werden. Für die Injektion bietet sich eine Formulierung in wässriger Lösung, vorzugsweise in physiologisch akzeptablen Puffern an (z.B. Hanks-Lösung, Ringer-Lösung oder physiologisch gepufferte Salzlösung). Für die parenterale Verabreichung, einschließlich der intravenösen, subkutanen, intramuskulären und intraperitonealen Verabreichung, kommt ebenfalls eine wässrige oder ölige Lösung oder eine Feststoffformulierung in Betracht. Der Anteil des aktiven Wirkstoffs in der pharmazeutischen Zusammensetzung kann variieren und liegt üblicherweise zwischen 2 und 60 Gew.-% der Verabreichungseinheit. Der Wirkstoffanteil ist entsprechend so ausgewählt, dass eine wirksame Dosis erreicht wird. Gemäß einer bevorzugten Ausführungsform der Erfindung wird das Delphinidin oder dessen Salze und/oder der Komplex aus Delphinidin und dem Sulfoalkylether-β-Cyclodextrin in einer galenischen Zubereitung zur kontrollierten und/oder zeitverzögerten Freisetzung des Delphinidins eingesetzt.

"Salz" oder "pharmazeutisch akzeptables Salz" steht für jegliches unter pharmazeutischen Gesichtspunkten akzeptables Salz einer Verbindung vorliegender Erfindung, welches den pharmazeutisch wirksamen Wirkstoff oder dessen aktives Metabolit nach der Verabreichung freigeben kann. Salze der Zusammensetzungen und Komplexe der vorliegenden Erfindung können von anorganischen oder organischen Säuren und Basen abgeleitet sein.

Das Anthocyanidin Delphinidin kann in "Reinform" oder "gereinigt" verwendet werden, was bedeutet, dass ungewünschte Komponenten entfernt wurden.

"Anthocyanidine" weisen die nachfolgend wiedergegebene Grundstruktur auf.

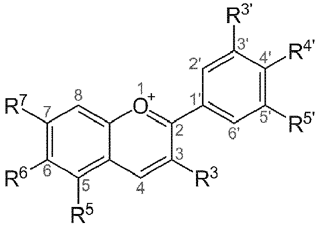

Die Substituenten in dieser Formel sind ausgewählt aus der Gruppe bestehend aus Wasserstoff, Hydroxygruppe und Methoxygruppe.

Cyclodextrine, die mit dem Anthocyanidin Delphinidin erfindungsgemäß komplexiert sein können, sind zyklische Oligosaccharide aus α-1,4-glykosidisch verknüpften Glukosemolekülen. ß-Cyclodextrin besitzt sieben Glukoseeinheiten. Bei einem Sulfoalkyether-ß-Cyclodextrin sind Hydroxygruppen der Glukoseeinheit in einem Sulfoalkylalkohol verethert. Erfindungsgemäß ist in der Regel lediglich ein Teil der 21 Hydroxygruppen eines ß-Cyclodextrins verethert. Die Herstellung von Sulfoalkyethercyclodextrinen ist dem Fachmann geläufig und beispielsweise in US 5,134,127 oder WO 2009/134347 A2 beschrieben.

Sulfoalkyethergruppen werden bei Cyclodextrinen im Stand der Technik zur Erhöhung der Hydrophilie beziehungsweise Wasserlöslichkeit eingesetzt. Sulfoalkylethergruppen tragen in besonderem Maße zur Erhöhung der Stabilität des Komplexes aus Anthocyanidinen und dementsprechend substituiertem ß-Cyclodextrin bei, so dass damit die Lagerstabilität und Formulierbarkeit der besonders oxidationsempfindlichen Anthocyanidine wesentlich verbessert wird. Der erfindungsgemäße Komplex kann als lagerstabile wässrige Lösung oder Feststoff formuliert werden, wie unten noch näher gezeigt werden wird.

Erfindungsgemäß besonders bevorzugt ist die Komplexierung des Wirkstoffs Delphindin mit einem Sulfoethylether-ß-Cyclodextrin, was überraschenderweise die Löslichkeit und Stabilität des Wirkstoffs erhöht. Ein den Schutzbereich nicht beschränkender Erklärungsversuch hierfür ist, dass die negativ geladenen Sulfoethyleinheiten mit den positiv geladenen Anthocyanidin Delphinidin elektrostatisch wechselwirken und unter den Alkylgruppen die Ethylgruppe die optimale Länge besitzt, um sterisch eine entsprechende Wechselwirkung zu ermöglichen. An dieser Stelle sei angemerkt, dass keine allgemein gültige Aussage getroffen werden kann, dass ein beliebiger Wirkstoff, beispielsweise Delphinidin, im Komplex mit einem Sulfoalkylether-β-Cyclodextrin zu einer Verbesserung der Löslichkeit und Stabilität führt. Beispielhaft verwiesen sei an dieser Stelle auf Tabelle 1 in Ueda et al., "Evaluation of a Sulfobutyl Ether β-Cyclodextrin as a Solubizing/Stabilizing Agent for Several Drugs", Drug Development and Industrial Pharmacy, 24(9), 863-867 (1998), in der die Löslichkeiten verschiedener Wirkstoffe allein, im Komplex mit Sulfobotylether-β-Cyclodextrin und im Komplex mit β-Cyclodextrin gegenübergestellt sind. Aus den dort dargestellten Löslichkeitswerten (SBE7-β-CD vs. β-CD) erkennt man, dass bei einem Drittel der untersuchten Wirkstoffe im Komplex mit Solfobutylether-β-Cyclodextrin genau das Gegenteil der Fall ist, d.h. der Komplex aus Wirkstoff und Sulfobutylether-β-Cyclodextrin eine signifikant geringere Löslichkeit gegenüber dem Komplex mit β-Cyclodextrin zur Folge hat.

Bevorzugt beträgt der Substitutionsgrad des Cyclodextrins mit Sulfoalkylethergruppen 3 bis 8, weiter vorzugsweise 4 bis 8, weiter vorzugsweise 5 bis 8, weiter vorzugsweise 6 bis 7. Ebenfalls verwendbar sind Sulfobutylether-β-Cyclodextrine mit gleichem Substitutionsgrad, so sind entsprechende Cyclodextrine mit einem mittleren Substitutionsgrad von 6 bis 7 beispielsweise in der genannten WO 2009/134347 A2 beschrieben und unter dem Handelsnamen Captisol® kommerziell erhältlich. Ebenfalls verwendbar sind entsprechende Cyclodextrine mit einem Substitutionsgrad von 4 bis 5, beispielsweise 4,2.

Das erfindungsgemäß in reiner, Salz- oder komplexierter Form verwendete Anthocyanidin ist Delphinidin. Die chemische Struktur entspricht der oben wiedergegebenen Formel mit dem folgenden Substitutionsmuster

| | R^{3'} | R^{4'} | R^{5'} | R³ | R⁵ | R⁶ | R⁷ |
|---|---|---|---|---|---|---|---|
| Delphinidin | -OH | -OH | -OH | -OH | -OH | -H | -OH |

Gegenstand der Erfindung ist ferner eine wässrige Lösung der erfindungsgemäßen Zusammensetzung zur Verwendung als Medikament gemäß den Ansprüchen.

Die Herstellung des erfindungsgemäßen Komplexes sowie einer entsprechend wässrigen Lösung umfasst die folgenden Schritte:
a) Herstellen einer wässrigen Lösung des Sufoalkylether-β-Cyclodextrins,
b) Zugabe des Anthocyanidin Delphinidin und Vermischen zur Herstellung des Komplexes.

In Schritt a) wird bevorzugt eine wässrige Lösung hergestellt, die 5 bis 10 Gew.-% des verwendeten Cyclodextrins enthält. Besonders bevorzugt ist es im Rahmen der Erfindung, wenn der pH-Wert der wässrigen Lösung während oder nach, bevorzugt jedoch vor der Zugabe des Delphinidins auf einen pH-Wert von 7 oder weniger, vorzugsweise 6 oder weniger, weiter vorzugsweise 5 oder weniger, weiter vorzugsweise 4 bis 5, eingestellt wird. Es hat sich gezeigt, dass bei diesem pH-Wert sich eine höhere Konzentration des Komplexes in wässriger Lösung einstellen lässt.

Die Konzentration des Delphinidins berechnet als Chlorid, beträgt vorzugsweise wenigstens 0,5 mg/ml, weiter vorzugsweise wenigstens 1,0 mg/ml, weiter vorzugsweise wenigstens 1,5 mg/ml, weiter vorzugsweise 2,0 mg/ml. Der besonders bevorzugte Konzentrationsbereich von wenigstens 2,0 mg/ml lässt sich im Rahmen einer bevorzugten Ausführungsform insbesondere einstellen in einer wässrigen Lösung mit einem pH-Wert zwischen 4 und 5.

Im Rahmen der Herstellung kann das Vermischen der Bestandteile der wässrigen Lösung durch Rühren geschehen, bevorzugte Zeiträume für das Vermischen sind 2 bis 20 h. Bevorzugt wird im Dunkeln gearbeitet, um eine lichtinduzierte Oxidation zu vermeiden.

Ein weiterer Gegenstand der Erfindung ist ein Feststoff zur Verwendung als Medikament, der erfindungsgemäß erhältlich ist durch Entfernen des Lösungsmittels aus einer oben beschriebenen erfindungsgemäßen wässrigen Lösung. Das Entfernen kann bevorzugt durch Gefriertrocknung (Lyophilisation) erfolgen. Sowohl die erfindungsgemäße wässrige medikamentöse Lösung als auch der medikamentöse Feststoff besitzen eine hohe Lagerstabilität.

Die Erfindung soll nun im Folgenden weiter in den Beispielen unter Bezug auf die beigefügten Figuren beschrieben werden, ohne auf diese beschränkt zu sein.
- Figur 1: zeigt das *in-vitro* Wirkungsprofil der prophylaktischen und/oder mit Lipopolysaccharid (LPS) gleichzeitigen Verabreichung von Delphinidin auf Zellen im Vergleich zu nur mit LPS (Kontrolle) behandelten Zellen.
- Figur 2a: zeigt das *in-vitro* Wirkungsprofil der mit Lipopolysaccharid (LPS) gleichzeitigen Verabreichung von Delphinidin oder Hydrocortison auf Zellen im Vergleich zu nur mit LPS (Kontrolle) behandelten Zellen.
- Figur 2b: zeigt das *in-vitro* Wirkungsprofil der mit Lipopolysaccharid (LPS) gleichzeitigen Verabreichung von Delphinidin auf Zellen im Vergleich zu nur mit LPS (Kontrolle) behandelten Zellen.
- Figur 3: zeigt das *in-vitro* Wirkungsprofil von Delphinidin auf Zellen bei Verwendung verschiedener Delphinidinkonzentration im Vergleich zu mit Hydrocortison behandelten Zellen.
- Figur 4: zeigt den Einfluss von Delphinidin und Dexamethason auf die mRNA Expression von Ccl7 [Chemokine (C-C motif) ligand 7] in cEND Zellen nach TNFα [Tumor Necrosis Factor α] Behandlung bzw. unbehandelten Zellen *in vitro.*
- Figur 5: zeigt den Einfluss von Delphinidin auf die mRNA Expression des Tight junction Proteins Claudin-5 in cEND Zellen nach TNFα [Tumor Necrosis Factor α] Behandlung bzw. unbehandelten Zellen *in vitro.*
- Figur 6: zeigt den *in-vivo* Einfluss von intraplantar verabreichten Delphinidin-Sulfoethylether-β-Cyclodextrin auf eine CFA (Complete Freund's Adjuvant)-induzierte Entzündung der Rattenhinterpfote.
- Figur 7: zeigt das in Beispiel 10 angewendete akute in vivo Maus-Herzinfarktmodell mit Zeitverlauf und den betroffenen Herzbereichen.
- Figur 8: zeigt Löslichkeiten von DelphinidinSBECD.
- Figur 9: zeigt das Versuchsprotokoll der Anwendung von DelphinidinSBECD in dem Modell gemäß Figur 7.
- Figur 10: zeigt die Herzinfarktgröße als Prozentwert des ischämischen Bereichs für DelphinidinSBECD im Vergleich zu der Kontrollgruppe.

### Beispiele

### I. Herstellung eines Komplexes aus Delphinidin und Cyclodextrinen

### 1. Eingesetzte Materialien:

Die nachfolgenden Cyclodextrine werden verwendet:

| | |
|---|---|
| α-CD | ID No: CYL-2322 |
| β-CD | ID No: CYL-3190 |
| γ-CD | ID No: CYL-2323 |
| (2-Hydroxypropyl)-ß-CD | ID No: L-043/07 |
| Sulfobutylether-ß-CD | ID No: 47K010111 |

Delphinidinchlorid wurde von der Firma Extrasynthese erworben.

### 2. Bestimmung des Delphinidingehalts

Zur Bestimmung des Gehalts von Delphinidinchlorid in den delphinidinhaltigen Zusammensetzungen wurde ein Reversephase HPLC-Verfahren verwendet. Dabei wurden folgende Reagenzien eingesetzt:
Gereinigtes Wasser
Methanol für die Chromatographie
Ameisensäure, p. a.
1 M Salzsäure als volumetrische Lösung.

Als Säule wurde eine Waters X Bridge™ C18,35 µl, 150 mm x 4,6 mm verwendet.

Die mobilen Phasen waren wie folgt:
Kanal A: Wasser 950 ml, Methanol 50 ml, Ameisensäure 10 ml
Kanal B: Wasser 50 ml, Methanol 950 ml, Ameisensäure 10 ml

Folgendes Gradientenprogramm wurde verwendet:

| Zeit [min] | Prozent Kanal B |
|---|---|
| 0 | 0 |
| 5 | 0 |
| 25 | 60 |
| 30 | 100 |

Stoppzeit: 35 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche
Lösungen und Probenvorbereitung:

| | |
|---|---|
| Verdünnungslösung 1: | Mischung aus 100 ml Methanol und 2,6 ml M HCL |
| Verdünnungslösung 2: | Mischung aus 100 ml 40 prozentiger Methanol und 2,6 ml 1 M HCL |

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts erfindungsgemäß hergestellter Feststoffe (Herstellung siehe weiter unten) wurden etwa 50 mg dieser Zusammensetzung in einem 10 ml Kolben eingewogen. Anschließend wurde in Verdünnungslösung 2 gelöst und mit der gleichen Verdünnungslösung 2 weiter verdünnt bis zur Einstellung einer ungefähren Delphinidinkonzentration von 0,1 mg/ml.

Die Bestimmung des Delphinidingehalts in den Proben wurde berechnet unter Zuhilfenahme der Agilent ChemStation Software unter Verwendung der Kalibrierung mit dem beschriebenen externen Standard.

### Beispiel 1: Komplexierung von Delphinidin mit SBE-ß-CD

In diesem Beispiel wird die Komplexierung von Delphinidin durch verschiedene Cyklodextrine und die Löslichkeit des Komplexes in wässriger Lösung untersucht.

Es wurden neutrale wässrige Lösungen hergestellt, die 10 Gew.-% des jeweiligen Cyclodextrines enthalten. Bei ß-CD wurde auf Grund der mangelnden Löslichkeit eine Konzentration von lediglich 2 Gew.-% gewählt.

Je 5 ml der wässrigen Cyclodextrinlösungen und von reinem Wasser wurden in Glaskolben gefüllt. Anschließend wurde ein Überschuss Delphinidinchlorid zugegeben. Die erforderliche Überschussmenge war 10 mg für die Lösungen von α-, ß- und γ-Cyclodextrin und 15 mg für die Lösungen von HPBCD (2-Hydroxypropyl-ß-Cyclodextrin) und SBE-ß-CD.

Die Suspensionen wurden für 20 h bei 30° C im Dunkeln gerührt. Anschließend wurde filtriert durch einen Membranfilter mit 0,22 µm Porengröße.

Die erzielbaren Löslichkeiten sind in der nachfolgenden Tabelle 1 wiedergegeben.

| Cyclodextrin | Cyclodextrin-Konzentration | Delphinidinchlorid |
|---|---|---|
| - | 0 | 0.07 mg/ml |
| α-CD | 10 % | 0.14 mg/ml |
| ß-CD | 2 % | 0.05 mg/ml |
| γ-CD | 10 % | 0.21 mg/ml |
| HPBCD | 10 % | 0.19 mg/ml |
| SBE-ß-CD | 10 % | 0.66 mg/ml |

Man erkennt, dass die Komplexierung und die dadurch bewirkte Löslichkeitserhöhung für SBE-ß-CD weit besser ist als für die anderen Cyclodextrine.

### Beispiel 2: Einfluss des pH-Wertes

In diesem Beispiel wurde der Einfluss des pH-Wertes auf die Löslichkeit eines Delphinidin-SBE-ß-CD in wässriger Lösung untersucht. Nach der Vorschrift von Beispiel 1 wurden wässrige Lösungen von SEB-ß-CD hergestellt, diese Lösungen jedoch mit 1 M HCL auf die in Tabelle 2 genannten sauren pH-Werte eingestellt. Anschließend wurde Delphinidinchlorid nach der Vorschrift des Beispiels 1 zugegeben und weitergearbeitet, als einzige Abweichung wurde die Rührzeit auf 2,5 h begrenzt. Die Ergebnisse sind in der nachfolgenden Tabelle 2 wiedergegeben.

| pH | Delphinidinchlorid |
|---|---|
| 6.0 | 0.60 mg/ml |
| 4.8 | 2.12 mg/ml |
| 4.1 | 2.03 mg/ml |

Man erkennt, dass bei pH-Werten zwischen 4 und 5 die Löslichkeit des komplexierten Delphinidinchlorids sich etwa um den Faktor 3 gegenüber einem neutralen pH-Wert erhöht.

### Beispiel 3 Herstellung eines erfindungsgemäßen Feststoffs

In diesem Beispiel wird ein erfindungsgemäßer Komplex als Feststoff formuliert. Zu Vergleichszwecken werden ein Delphinidin/HPBCD Komplex sowie eine Delphinidin/Stärkeformulierung als Festsoff zubereitet.

### Beispiel 3.1: Delphinidin/SBE-ß-CD

5 g SEB-ß-CD wurden in 40 ml destilliertem Wasser zu einer klaren Lösung gelöst. Der pH-Wert der Lösung wurde mittels 1 M HCL auf 4,8 eingestellt. Anschließend wurden 0,11 g Delphinidinchlorid hinzugefügt und 2 h bei 27°C im Dunkeln gerührt. Die homogene Flüssigkeit wurde durch einen Cellulosenitratmembranfilter mit einer Porengröße von 0,45 µm vakuumfiltriert. Die Lösung wurde gefroren und anschließend gefriergetrocknet bei -48°C und einem Druck von etwa 10,3 Pa (77 mTorr). Das Lyophilisat wurde gemahlen und durch ein Sieb von 0,3 mm Maschenweite gesiebt.

### Beispiel 3.2: Delphinidin/HPBCD

Es wurde in gleicher Weise wie Beispiel 3.1 gearbeitet, jedoch wurde bei der Filtration eine signifikante Menge Material abfiltriert, was darauf hindeutet, dass die Solubilisierung deutlich weniger effektiv war als bei Verwendung von SBE-ß-CD gemäß Beispiel 3.1.

### Beispiel 3.3 Delphinidin/Stärkeformulierung

5 g Stärke wurde in 40 ml destilliertem Wasser suspendiert. Man erhielt eine weiße Suspension. Der pH-Wert der Lösung wurde mit 1 M HCL auf 4,6 eingestellt. Anschließend wurde 0,11 g Delphinidinchlorid zugegeben und für 2 h bei 27° C im Dunkeln gerührt. Die erhaltene homogene Flüssigkeit wurde wie in Beipiel 3.1 gefriergetrocknet, gemahlen und gesiebt.

Beispiel 3.1 ist erfindungsgemäß, bei den Beispielen 3.2 und 3.3 handelt es sich um Vergleichsbeipiele.

### Beispiel 4: Stabilitätsversuche

Die Feststoffe gemäß den Beipielen 3.1 bis 3.3 wurden unter folgenden Bedingungen gelagert:
- 8 Tage bei Raumtemperatur in braunen, verschraubten Glasbehältern,
- anschließend 22 Tage bei Raumtemperatur in Glasbehältern unter Sauerstoffatmosphäre im Dunkeln.

Die letzen 22 Tage der oben beschriebenen Lagerung wurden in Glasviolen mit einem Volumen von 20 ml durchgeführt. Jeweils 250 mg der vorher bereits für 8 Tage gelagerten Proben wurden dort eingefüllt, die Violen wurden mit einem Gummistopfen verschlossen und abgedichtet. Mittels zwei Injektionsnadeln wurde der Kopfraum der Violen mit reinem Sauerstoff gespült. Die Proben wurden anschließend im Dunkeln gelagert.

Der Delphinidingehalt der Feststoffe (berechnet als Delphinidinchlorid und angegeben in Gew.-%) wurde mittels der oben beschriebenen HPLC-Methode bestimmt. Die Ergebnisse finden sich in der nachfolgenden Tabelle 3.

| | Zeitablauf [Tage] | | | | |
|---|---|---|---|---|---|
| | Start | 2 | 8 | 19 | 30 |
| Beispiel 3.1 | 1.69 | 1.52 | 1.55 | 1.40 | 0.93 |
| Beispiel 3.2 | 1.30 | 1.20 | 1.14 | 1.03 | 0.68 |
| Beispiel 3.3 | 1.60 | 1.59 | 1.56 | 1.53 | 1.15 |

Die Ergebnisse zeigen, dass sich erfindungsgemäß ein Delpinidinkomplex herstellen lässt, der selbst unter reiner Sauerstoffatmosphäre eine hohe Stabilität und damit gute Lagerfähigkeit besitzt. Der Komplex besitzt ferner eine gute Löslichkeit in wässrigen, insbesondere leicht sauren Lösungen, sodass sich Delphinidin erfindungsgemäß auf vielfältige Art und Weise formulieren lässt. Die Stabilität des erfindungsgemäßen Feststoffes ist ähnlich gut wie eine Formulierung mit Stärke (Beispiel 3.3), dieses Vergleichsbeispiel lässt sich allerdings nicht in einer wässrigen Lösung formulieren.

### Beispiel 5: Stabilitätsversuche in wässriger Lösung

Zur Bestimmung des Gehalts von Delphinidinchlorid in den delphinidinhaltigen Lösungen wurde ein Reverse Phase HPLC-Verfahren ähnlich dem oben bereits beschriebenen verwendet. Dabei wurden folgende Reagenzien eingesetzt:
Gereinigtes Wasser
Methanol für die Chromatographie
Armeisensäure, p. a.
1 M Salzsäure als volumetrische Lösung.

Als Säule wurde eine Waters X Bridge™ C18, 35 µl, 150 mm x 4,6 mm verwendet.

Die mobilen Phasen waren wie folgt:
Kanal A: Wasser 770 ml, Methanol 230 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 20 | 20 |
| 25 | 100 |

Stoppzeit: 25 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche
Lösungen und Probenvorbereitung:

| | |
|---|---|
| Verdünnungslösung 1: | Mischung aus 100 ml Methanol und 2,6 ml 1 M HCL |
| Verdünnungslösung 2: | Mischung aus 100 ml 50 %igem Methanol und 2,6 ml 1 M HCL |

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts einer erfindungsgemäßen wässrigen Lösung wurden Delphinidin/SBE-ß-CD des Beispiels 3.1 (erfindungsgemäß) und Delphinidin (Vergleichsbeispiel in 0,9 %iger NaCl-Lösung gelöst bis zur Einstellung einer Startkonzentration (bezogen auf das Delphinidin) von 1,584 mg/ml (erfindungsgemäßes Beispiel) bzw. 0,0216 mg/ml (Vergleichsbeispiel). Die Lösungen wurden bei Raumtemperatur hergestellt und anschließend bei 37°C im Dunkeln in verschlossenen Violen gelagert.

Nach 1, 2, 3 und 4 h wurde der Delphinidingehalt bestimmt. Die nachfolgende Tabelle gibt den ermittelten Gehalt als Prozentanteil der oben genannten Startkonzentration an.

| Zeit [h] | Delphinidin unkomplexiert | Delphinidin/SBE-ß-CD |
|---|---|---|
| 0 | 100% | 100% |
| 1 | 8,3% | 80,7% |
| 2 | 6,5% | 74,5% |
| 3 | 5,6% | 64,7% |
| 4 | 5,1% | 62,8% |

Die Bestimmung des Delphinidingehalts in den Proben wurde berechnet unter Zuhilfenahme der Agilent ChemStation Software unter Verwendung der Kalibrierung mit den beschriebenen externen Standard.

### II. Antiphlogistische und antiinflammatorische Wirkung von Delphinidin und Delphinidin-Sulfoethylether-β-Cyclodextrin in-vitro und in-vivo

### Beispiel 6: In-vitro Wirkungsprofil der prophylaktischen und/oder mit Lipopolysaccharid (LPS) gleichzeitigen Verabreichung von Delphinidin auf Zellen im Vergleich zu nur mit LPS (Kontrolle) behandelten Zellen.

### Methodik

Bei gesunden Probanden wurde mittels Immunfluoreszenz Kernfärbung für NFKB die Konzentration von NFKB im Zellkern von Monozyten nach Stimulation mit LPS und/oder Delphinidin getestet und im Vergleich zu nicht stimulierten Zellen ins Verhältnis gesetzt. NFKB wird durch unterschiedliche, vor allem inflammatorische und stress-assoziierte Stimuli aktiviert. Inaktive NFKB-Proteine sind im Zytosol an Inhibitor-Proteine der IKB-Familie gebunden (Baeuerle and Baltimore, 1988, Activation of DNA-binding activity in an apparently cytoplasmic precursor of the NF-kappa B transcription factor, Cell (53), 211-7; Baeuerle and Baltimore, 1988, I kappa B: a specific inhibitor of the NF-kappa B transcription factor, Science (242), 540-6), wobei die Stimulus-abhängige proteolytische Degradation verschiedener IKB-Mitglieder die Kernwanderung von NFKB bewirkt (Ghosh et al., 1988, NF-kappa B and Rel proteins: evolutionarily conserved mediators of immune responses, Annu Rev Immunol (16), 225-60) und somit die Bestimmung das Ausmaßes der Translokation von NFKB aus dem Zytosol in den Zellkern ein geeignetes Nachweismittel für die inflammatorische bzw. antiinflammatorische Wirkung zu untersuchender Zusammensetzungen ist.

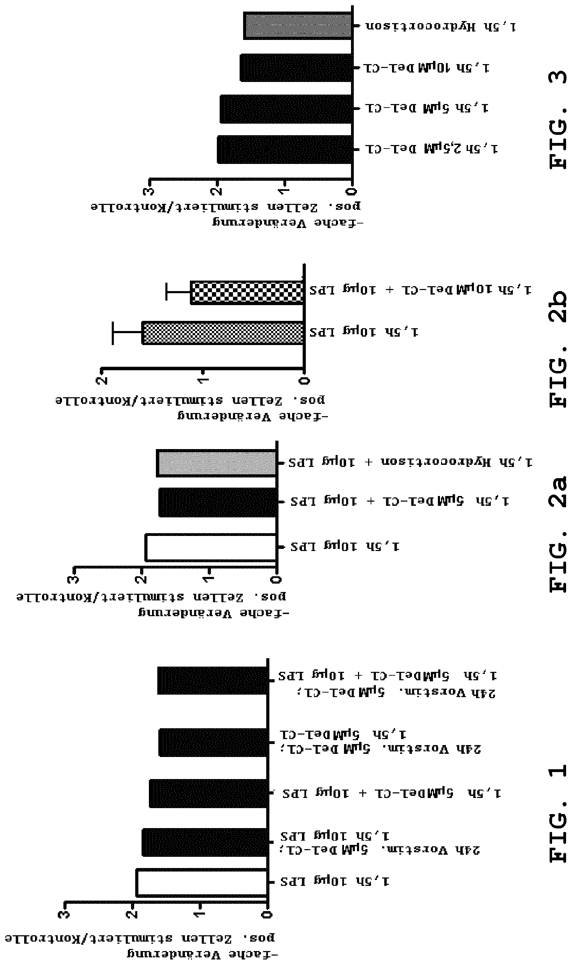

Es wurde wie folgt vorgegangen und in Figur 1 von links nach rechts dargestellt, wobei die Stimualtion/Inkubation der Zellen (jeweils 1 ml RPMI-1640 umfassend 1 Million Zellen in der exponentiellen Wachstumsphase) in einer Vertiefung einer 24-Well-Polysterol-Zellkulturschale bei 37°C erfolgte und die angegebenen Konzentrationen Endkonzentrationen sind:

| | |
|---|---|
| "1,5h 10µg LPS" | - Stimulation der Zellen 1,5 h mit 10µg/ml LPS (Kontrolle) |
| "24h Vorstim. 5µM Del-Cl; 1,5h 10µg LPS" | - 24h Vorstimulation der Zellen mit 5µM Delphinidinchlorid (gelöst in RPMI- 1640 Zellmedium) und anschließend 1,5h Stimulation mit 10µg/ml LPS |
| "1,5h 5µM Del-Cl + 10µg LPS" | - 1,5h Stimulation der Zellen mit 10µg/ml LPS und 5µM Delphinidinchlorid (gelöst in RPMI-1640 Zellmedium) |
| "24h Vorstim. 5µM Del-Cl; 1,5h 5µM Del-Cl" | - 24h Vorstimulation der Zellen mit 5µM Delphinidinchlorid (gelöst in RPMI- 1640 Zellmedium) und anschließend er neute Stimulation mit 5µM Delphini dinchlorid (gelöst in RPMI-1640 Zell medium) für 1,5h |
| "24h Vorstim. 5µM Del-Cl; 1,5h 5µM Del-Cl + 10µg LPS" | - 24h Vorstimulation der Zellen mit 5µM Delphinidinchlorid (gelöst in RPMI- 1640 Zellmedium) und anschließend erneute Stimulation mit 5µM Delphini dinchlorid (gelöst in RPMI-1640 Zell medium) sowie 10µg/ml LPS für 1,5h |

### Ergebnisse

Wie aus Figur 1 ersichtlich hemmt eine prophylaktische Gabe von 5µM Delphinidin 24h vor der Stimulation der Zellimmunantwort mit LPS (schwach) die nachfolgende 1,5h LPS-vermittelte Immunantwort("24h Vorstim. 5µM Del-Cl; 1,5h 10µg LPS" versus "1,5h 10µg LPS"), die Hemmung der Immunantwort ist geringer als bei gleichzeitiger Gabe von Delphinidin in Kombination mit LPS ("1,5h 5µM Del-Cl + 10µg LPS"), und kann durch prophylaktische und wiederholte Zugabe von 5µM Delphinidin bei der LPS-Zugabe "24h Vorstim. 5µM Del-Cl; 1,5h 5µM Del-Cl + 10µg LPS") weiter verstärkt werden (additiver Effekt).

### Beispiel 7: In-vitro Wirkungsprofil der mit Lipopolysaccharid (LPS) gleichzeitigen Verabreichung von Delphinidin oder Hydrocortison auf Zellen im Vergleich zu nur mit LPS (Kontrolle) behandelten Zellen.

### Methodik

Es wurde analog der Methodik in Beispiel 5 vorgegangen und in den Figuren 2a und 2b von links nach rechts dargestellt, wobei die Stimualtion/Inkubation der Zellen (jeweils 1 ml RPMI-1640 umfassend 1 Million Zellen in der exponentiellen Wachstumsphase) in einer Vertiefung einer 24-Well-Polysterol-Zellkulturschale bei 37°C erfolgte und die angegebenen Konzentrationen Endkonzentrationen sind:

| | |
|---|---|
| Figur 2a: | |
| "1,5h 10µg LPS" | - Stimulation der Zellen 1,5 h mit 10µg/ml LPS (Kontrolle) |
| "1,5h 5µM Del-Cl + 10µg LPS" | - 1,5h Stimulation der Zellen mit 10µg/ml LPS und 5µM Delphinidinchlorid (gelöst in RPMI-1640 Zellmedium) |
| "1,5h Hydrocortison + 10µg LPS" | - Stimulation der Zellen mit 10⁻⁵M Hydro cortison und 10µg/ml LPS für 1,5h |
| Figur 2b: | |
| "1,5h 10µg LPS" | - Stimulation der Zellen 1,5 h mit 10µg/ml LPS (Kontrolle) |
| "1,5h 10µM Del-Cl + 10µg LPS" | - 1,5h Stimulation der Zellen mit 10µg/ml LPS und 10µM Delphinidinchlorid (ge löst in RPMI-1640 Zellmedium) |

### Ergebnisse

Wie aus Figur 2a ersichtlich wird die LPS-vermittelte Immunantwort der Zellen bei gleichzeitiger Gabe von 5µM Delphinidin ("1,5h 5µM Del-Cl + 10µg LPS") mindestens genauso gut gehemmt, wie durch Zugabe von vergleichsweise herangezogenem Hydrocortison ("1,5h Hydrocortison + 10µg LPS"). In einem in Figur 2b dargestellten Widerholungsversuch wurde bestätigt, dass die LPS-vermittelte Immunantwort der Zellen bei gleichzeitiger Gabe von 10 µM Delphinidin ("1,5h 10µM Del-Cl + 10µg LPS") signifikant verringert ist.

### Beispiel 8: In-vitro Wirkungsprofil von Delphinidin auf Zellen bei Verwendung verschiedener Delphinidinkonzentration im Vergleich zu mit Hydrocortison behandelten Zellen.

### Methodik

Es wurde analog der Methodik in den Beispielen 5 und 6 vorgegangen und in Figur 3 von links nach rechts dargestellt, wobei die Stimualtion/Inkubation der Zellen (jeweils 1 ml RPMI-1640 umfassend 1 Million Zellen in der exponentiellen Wachstumsphase) in einer Vertiefung einer 24-Well-Polysterol-Zellkulturschale bei 37°C erfolgte und die angegebenen Konzentrationen Endkonzentrationen sind:

| | |
|---|---|
| "1,5h 2,5µM Del-Cl" | - 1,5h Stimulation der Zellen mit 2,5µM Delphinidinchlorid (gelöst in RPMI-1640 Zellmedium) |
| "1,5h 5µM Del-Cl" | - 1,5h Stimulation der Zellen mit 5µM Delphinidinchlorid (gelöst in RPMI- 1640 Zellmedium) |
| "1,5h 10µM Del-Cl" | - 1,5h Stimulation der Zellen mit 10µM Delphinidinchlorid (gelöst in RPMI- 1640 Zellmedium) |
| "1,5h Hydrocortison" | - 1,5h Stimulation der Zellen mit 10⁻ ⁵M Hydrocortison |

### Ergebnisse

Wie aus Figur 3 ersichtlich nimmt mit steigender Delphinidinkonzentration auch die Delphinidin vermittelte Hemmung der Zellimmunantwort zu (Dosiseffekt), wobei die hemmende Wirkung bei entsprechend hoher Konzentration mit der von Hydrocortison vergleichbar ist.

### Beispiel 9: Zellprotektive (antiinflammatorische/ antiphlogistische) Wirkung von Delphinidin auf die Blut-Hirn-Schranke, insbesondere Einfluss von Delphinidin im Vergleich zu Dexamethason auf die mRNA Expression von Ccl7 [Chemokine (C-C motif) ligand 7] in cEND Zellen nach TNFα [Tumor Necrosis Factor α] Behandlung bzw. unbehandelten Zellen in vitro)

### Methodik

Um die antiinflammatorische Wirkung von Delphinidin zu untersuchen, wurde ein *in-vitro* Modell der Blut-Hirn-Schranke, cEND Zellen (Forster, C. et al., Occludin as direct target for glucocorticoid-induced improvement of blood-brain barrier properties in a murine in vitro system. J Physiol 565 (Pt 2), 475 (2005)), verwendet. Dieses *in-vitro* Modell besteht aus den mikrovaskulären Endothellzellen, die aus den Mäusehirnkapillaren isoliert und immortalisiert wurden. Die Zellen werden auf mit Kollagen IV (Bestandteil der Basallamina um die Hirnkapillaren) beschichtete Zellkulturgefäße ausgesät.

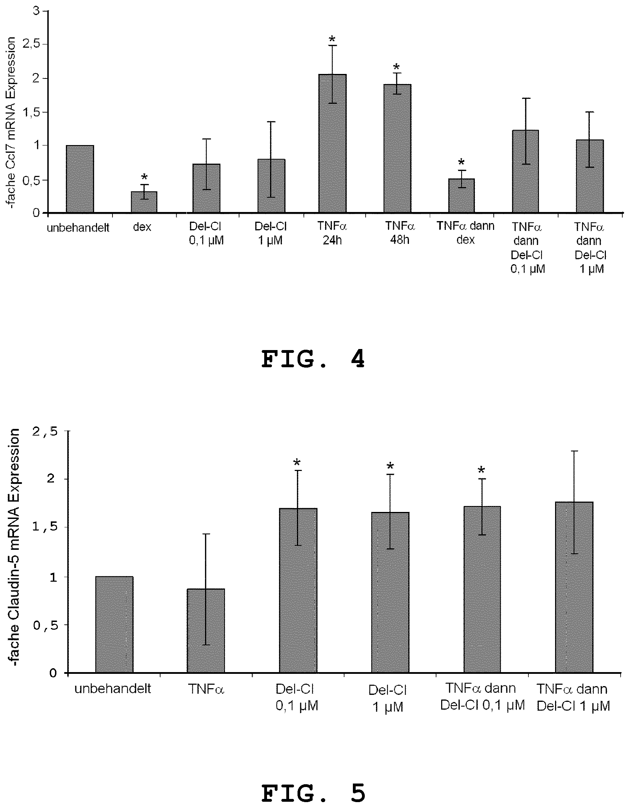

Exposition von cEND Zellen mit Tumor Nekrosis Factor alpha (TNFα) und Bestimmung der Ccl7 mRNA Expression als Marker für Inflammation. Als Referenzsubstanz für eine antiinflammatorische Wirkung wurde Dexamethason, ein synthetisches Steroidhormon mit starker antiinflammatorischer Wirkung, gewählt und die Wirkung von Delphinidin in Relation zur Wirkung von Dexamethason im in Figur 4 dargestellten Diagramm aufgetragen. Es folgten RNA Isolation und qPCR Untersuchungen vom pro-inflammatorischen Zytokin, Ccl7 (Chemokine (C-C motif) ligand 7), auch als Mcp-3 bekannt (monocyte chemotactic protein-3).

Es wurde wie folgt vorgegangen und in Figur 4 von links nach rechts dargestellt:

| | |
|---|---|
| "unbehandelt" | - Kontrolle ohne Reiz |
| "dex" | - Dexamethason |
| "Del-Cl 0,1µM" | - Delphindin 0,1µMol |
| "Del-Cl 1µM" | - Delphindin 0,1µMol |
| "TNFα 24h" | - Exposition mit TNFα für 24h |
| "TNFα 48h" | - Exposition mit TNFα für 48h |
| "TNFα dann dex" | - Exposition mit TNFα für 24h, anschließend Hinzufügen von Dexamethason und Expositi on für weitere 24h |
| "TNFα dann Del-Cl 0,1µM" | - Exposition mit TNFα für 24h, anschließend Hinzufügen von Delphinidin 0,1µMol und Exposition für weitere 24h |
| "TNFα dann Del-Cl 1µM" | - Exposition mit TNFα für 24h, anschließend Hinzufügen von Delphinidin 1µMol und Ex position für weitere 24h |

### Ergebnisse

Behandlung mit TNFα führte zum Anstieg der Ccl7 mRNA Expression auf das Zweifache im Vergleich zu unbehandelten Zellen. Zugabe von Delphinidin in beiden gewählten Konzentrationen von 0,1µM und 1µM führt zu einer deutlichen Unterdrückung bzw. Hemmung des TNFα-induzierten Ccl7 mRNA Expressionsanstiegs. Interessanterweise führte auch die Zugabe von Delphinidin allein in das Zellkulturmedium ohne vorheriger TNFα Exposition zur Suppression der Ccl7 Expression in den cEND Zellen. Ein starker Effekt konnte ebenfalls bei vergleichsweise herangezogenem Dexamethason nachgewiesen werden.

Aus den Messergebnissen lässt sich schlussfolgern, dass die antiinflammatorische Wirkung von Delphinidin auf die Blut-Hirn-Schranke nach Entzündung mindestens gleichwertig zu dem bisherigen "Goldstandard" Dexamethason ist.

### Beispiel 10: (Wirkung von Delphinidin auf die Barriereeigenschaften der Blut-Hirn-Schranke, insbesondere Einfluss von Delphinidin auf die mRNA Expression des Tight junction Proteins Claudin-5 in cEND Zellen nach TNFα [Tumor Necrosis Factor α] Behandlung bzw. unbehandelten Zellen in vitro)

### Methodik

Als Surrogat-Marker für die Barrieredichte wurde die Expression vom Tight junction Protein Claudin-5 verwendet (Forster C. Tight junctions and the modulation of barrier function in disease. Histochem Cell Biol. 2008; 130: 55-70) und analog Beispiel 8 vorgegangen.

### Ergebnisse

Die TNFα Behandlung führte zur Minderung der Claudin-5 mRNA Expression, wobei die Zugabe von Delphinidin nach 24h Stunden dieser TNFα-vermittelten Claudin-5 mRNA Expressionsminderung in beiden gewählten Konzentrationen (0,1µM und 1µM) entgegen wirkte, wie in Figur 5 dargestellt. Interessanterweise führte auch die Zugabe von Delphinidin allein in das Zellkulturmedium ohne vorherige TNFα Exposition zur Steigerung der Claudin-5 mRNA Expression.

Aus den Messergebnissen lässt sich schlussfolgern, dass die Barriere der Blut-Hirn-Schranke nach Entzündung unter dem Einfluss von Delphinidin stark zunimmt.

### Beispiel 11: In-vivo Einfluss von Delphinidin-Sulfoethylether-β-Cyclodextrin auf eine CFA (Complete Freund's Adjuvant)-induzierte Entzündung der Rattenhinterpfote.

### Methodik

### a) CFA-induzierte Entzündung der Rattenhinterpfote

CFA wird in einer Dosis von 150 µl intraplantar in die rechte hintere Rattenpfote unter Isoflurannarkose induziert. Die Entzündung und der darauffolgende Schmerz in der entzündeten Pfote entwickeln sich innerhalb 2 bis 96 h. Bis zu diesem Zeitpunkt gibt es keinen signifikanten Unterschied im Essverhalten, Körpergewicht, Kerntemperatur oder dem generellen Aktivitätslevel im Vergleich zu den nicht behandelten Tieren (Stein et al., Unilateral inflammation of the hindpaw in rats as a model of prolonged noxious stimulation: alterations in behavior and nociceptive thresholds, Pharmacol Biochem Behav., 1988; 31(2): 445-51)

### b) Verabreichung von H₂O (Kontrolle) und Delphinidin-Sulfoethylether-β-Cyclodextrin (Delphinidin-SEß CD)in unterschiedlichen Dosierungen

Den Ratten wird 96 Stunden nach vorgenanntem Schritt a) intraplantar in die rechte hintere Rattenpfote 100 µl H₂O (Kontrolle) bzw. 100 µl Delphinidin-SEßCD mit unterschiedlichen Dosierungen (2,50 mg/ml und 5,00 mg/ml) unter Isoflurannarkose injiziert.

### c) Algesiometrisches Messverfahren

Die Ratten wurden wie folgt an die entsprechende Versuchsbedingungen gewöhnt:
Für die Gewöhnung an den Randall-Sellito-Test wurden die Tiere 3 x pro Tag in den ersten drei Tagen locker unter einem Zellstoff gehalten, um sie an die Situation des Experimentes zu gewöhnen.

Am vierten Tag fand der oben genannte Behandlungsschritt b) statt, wobei beginnend vom Zeitpunkt 0 nach der Injektion nach 5, 15, 30, 60, 120 und 240 Minuten die Messung der Pfotendruckschwelle mittels Randall-Sellito-Test (Gerät von Ugo Basile) erfolgte.

Jede Pfote ipsilateral und kontralateral wird drei Mal im Abstand von mindestens 10s gemessen. Für den Randall-Sellito-Test wird die Ratte locker unter Zellstoff gehalten und die Pfote auf ein kleines Podest gesetzt. Dann wird von oben über einen Stempel ein steigender Druck auf die Pfote ausgeübt bis die Ratte die Pfote wegzieht (Stein et al., Intrinsic mechanisms of antinociception in inflammation: local opioid receptors and beta-endorphin, J Neurosci. 1990; 10(4): 1292-8; Rittner et al., Pain control by CXCR2 ligands through Ca2+-regulated release of opioid peptides from polymorphonuclear cells, FASEB J. 2006; 20(14): 2627-9).

### Ergebnisse

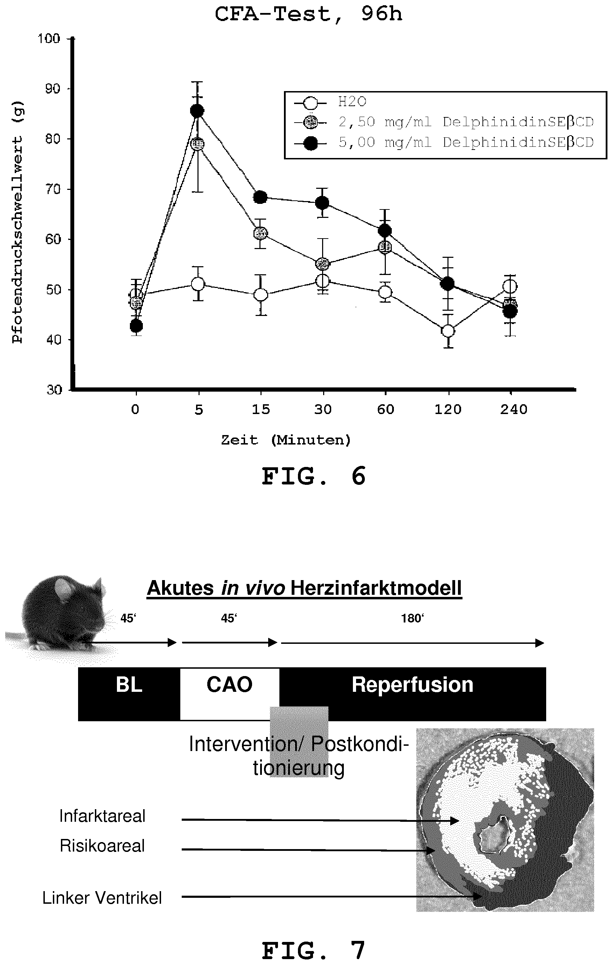

Auf den FAC-induzierten Entzündungsreiz in der Pfote der Ratten wurde deren mechanische Schmerzschwelle untersucht. Aus den ermittelten Pfotendruckschwellwerten, die in Figur 6 graphisch aufbereitet dargestellt sind, lässt sich ableiten, dass die Schmerzschwelle bei der Verabreichung von Delphinidin-SEßCD gegenüber der Kontrollinjektion mit H₂O signifikant reduziert ist, insbesondere in den ersten 120 Minuten, wobei die stärkste Wirkung bereits 5 Minuten nach der Verabreichung von Delphinidin-SEßCD auftritt. Dies gilt für beide gemessene Dosen von 2,5 mg/ml und 5,00 mg/ml Delphinidin-SEßCD, wobei sich aus der signifikant stärkeren Wirkung der höheren Konzentration in den Messpunkten der ersten 30 Minuten ebenfalls eine Dosisabhängigkeit ableiten lässt.

### Beispiel 12: Kardioprotektion durch Delphinidin-Sulfobothylether-β-Cyclodextrin (DelphinidinSBECD)

### Methodik und Ergebnisse

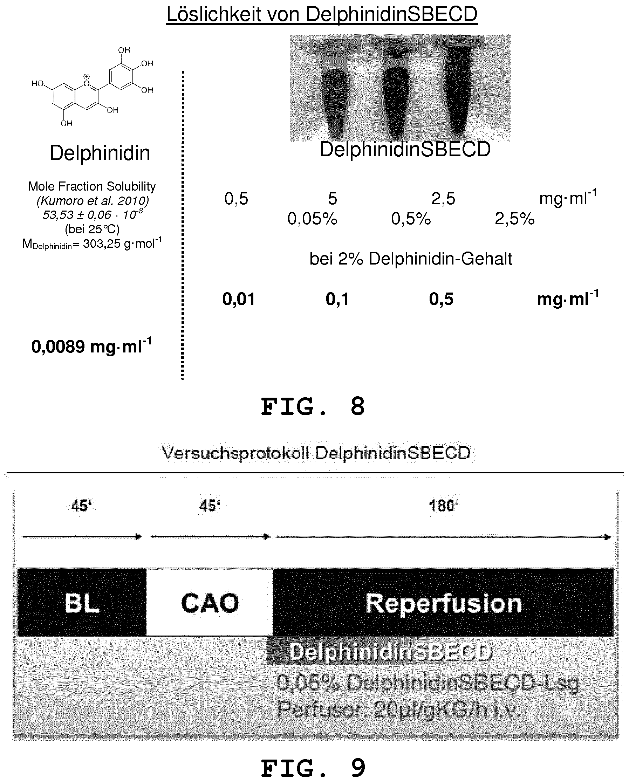

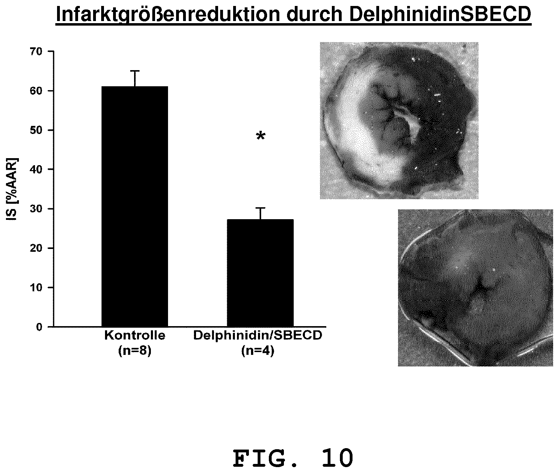

In Beispiel 11 wurde eine mögliche Postkonditionierungswirkung von DelphinidinSBECD im Vergleich zu einer Kontrollgruppe untersucht. Dazu wurde im *in vivo - in situ* Herzinfarktmodell der Maus mit den in den Figuren 7 und 9 dargestellten Zeitabläufen unter Anwendung der in Redel et *al.,* 2008 [Redel et al., Impact of Ischemia and Reperfusion Times on Myocardial Infarct Size in Mice In Vivo. Experimental Biology and Medicine (2008), 233:84-93] und Stumpner et *al.,* 2012 [Stumpner et al., Desflurane-induced postconditioning against myocardial infarction is mediated by calcium-activated potassium channels: role of the mitochondrial permeability transition pore. British Journal of Anaesthesia (2012), 108(4): 594-601] beschriebenen Methodik die resultierende Herzinfarktgröße bewertet und in Figure 10 graphisch dargestellt. Angegeben sind in Figur 10 Mittelwert±SEM; IS = Herzinfarktgröße; AAR = Risikobereich (area at risk) und *=signifikant verschieden von der Kontrollgruppe, wenn p<0,05. Die Daten sind als Mittelwert ± Standartfehler des Mittelwerts (SEM) dargestellt.

Aus den ermittelten IS- und %AAR-Werten, die in Figur 10 graphisch aufbereitet dargestellt sind, lässt sich ableiten, dass die Verabreichung von DelphinidinSBECD zu einer im Vergleich zur Kontrollgruppe signifikanten Infarktgrö-ßenreduktion führt.

## Patentansprüche

1. Zusammensetzung umfassend einen Komplex aus Delphinidin und einem Sulfoalkylether-β-Cyclodextrin zur Verwendung als Antiphlogistikum und/oder Immunsuppressivum.

2. Zusammensetzung zur Verwendung nach Anspruch 1, **dadurch gekennzeichnet, dass** die Verwendung die Gewebe- und/oder Organprotektion bei oder infolge zerebraler und/oder myokardialer Ischämie, vorzugsweise bei drohenden Reperfusionsschäden, umfasst.

3. Zusammensetzung zur Verwendung nach Anspruch 2, **dadurch gekennzeichnet, dass** die zerebrale Ischämie und die myokardiale Ischämie mit den klinischen Krankheitsbildern des Schlaganfalls und des Myokardinfarkts korrelieren.

4. Zusammensetzung zur Verwendung nach Anspruch 1, **dadurch gekennzeichnet, dass** die Verwendung die Prophylaxe und/oder Behandlung einer Indikation, ausgewählt aus der Gruppe bestehend aus
- entzündlichen Zuständen oder entzündlichen Erkrankungen,
- mit entzündlichen Gewebeveränderungen assoziierte Erkrankungen,
- Abstoßungsreaktionen nach einer Transplantation und
- Autoimmunerkrankungen
umfasst.

5. Zusammensetzung zur Verwendung nach Anspruch 4, **dadurch gekennzeichnet, dass** die entzündlichen Zustände oder entzündlichen Erkrankungen ausgewählt sind aus der Gruppe bestehend aus rheumatoide Arthritis, chronische Polyarthritis, juvenile idiopathische Arthritis,Spondylitis, Osteoarthritis, Sepsis, Septischer Schock, Zerebrale Malaria, eine chronisch entzündliche Lungenerkrankung, Silikose, Sarkoidose, Reperfusionssyndrom, inflammatorisch-neurodegenerative Erkrankungen, einschließlich Crohn'sche Erkrankung, Multiple Sklerose und Parkinson, Colitis ulzerosa, Depressionen und Fieber bei Infektionen.

6. Zusammensetzung zur Verwendung nach Anspruch 4, **dadurch gekennzeichnet, dass** die mit entzündlichen Gewebeveränderungen assoziierten Erkrankungen ausgewählt sind aus der Gruppe bestehend aus Alzheimer, Parkinson und Krebs.

7. Zusammensetzung zur Verwendung nach einem der vorangehenden Ansprüche, **dadurch gekennzeichnet, dass** das Sulfoalkylether-β-Cyclodextrin ein Sulfobutylether-β-Cyclodextrin oder ein Sulfoethylether-β-Cyclodextrin ist.

8. Zusammensetzung zur Verwendung nach einem der vorangehenden Ansprüche, **dadurch gekennzeichnet, dass** der Substitutionsgrad des Cyclodextrins mit Sulfoalkylethergruppen 3 bis 8, vorzugsweise 4 bis 8, weiter vorzugsweise 5 bis 8, weiter vorzugsweise 6 bis 7 beträgt.

9. Zusammensetzung zur Verwendung nach einem der vorangehenden Ansprüche, **dadurch gekennzeichnet, dass** die Zusammensetzung eine therapeutisch wirksame Menge des Komplexes aus Delphinidin und Sulfoalkylether-β-Cyclodextrin umfasst.

10. Zusammensetzung zur Verwendung nach einem der vorangehenden Ansprüche **dadurch gekennzeichnet, dass** die Zusammensetzung als Monopräparat verwendet wird.

11. Zusammensetzung zur Verwendung nach einem der Ansprüche 1-9, **dadurch gekennzeichnet, dass** die Zusammensetzung mindestens eine weitere therapeutisch aktive Substanz umfasst.

12. Zusammensetzung zur Verwendung nach Anspruch 11, **dadurch gekennzeichnet, dass** die mindestens eine weitere therapeutisch aktive Substanz ausgewählt ist aus der Gruppe bestehend aus der Gruppe der Antiphlogistika, der Antikörper gegen inflammatorische Zytokine, der löslichen Rezeptoren inflammatorischer Zytokine oder der Immunosuppressiva und vorzugsweise ausgewählt ist aus der Gruppe bestehend aus Acetylsalicylsäure, Diclophenac, Indometacin, Ciclosporin, Azathioprin, Bortezomib, Melphalan, Prednison, Vincristin, Carmustin, Cyclophosphamid, Dexamethason, Thalidomid, Doxorubicin, Cisplatin, Etoposid und Cytarabin.

13. Zusammensetzung zur Verwendung nach einem der vorangehenden Ansprüche, weiter umfassend ein oder mehrere pharmazeutische Hilfs- und/oder Zusatzstoffe, vorzugsweise ausgewählt aus der Gruppe bestehend aus einen pharmazeutisch akzeptablen Träger, Füllstoffe, Geruchsstoffe und Stabilisatoren.

14. Zusammensetzung zur Verwendung nach einem der vorangehenden Ansprüche in einer Formulierungsform zur Verabreichung in einer Form ausgewählt aus der Gruppe bestehend aus oral, rektal, parenteral, einschließlich intraperitoneal, perkutan, subkutan, intramuskulär, intravenös, ophthalmisch, pulmonal und nasal.

15. Zusammensetzung zur Verwendung nach Anspruch 14, **dadurch gekennzeichnet, dass** die Verabreichungsform ausgewählt ist aus der Gruppe bestehend aus Tablette, Kapsel, Suspension, Aerosol, Lösung, Creme, Paste, Lotion, Gel und Salbe.

16. Zusammensetzung zur Verwendung nach einem der vorangehenden Ansprüche, **dadurch gekennzeichnet, dass** der Komplex aus Delphinidin und dem Sulfoalkylether-β-Cyclodextrin in einer galenischen Zubereitung zur kontrollierten und/oder zeitverzögerten Freisetzung des Delphinidins eingesetzt wird.

## Claims

1. Composition comprising a complex composed of delphinidin and a sulfoalkyl ether β-cyclodextrin for use as an antiinflammatory and/or immunosuppressant.

2. Composition for the use according to Claim 1, **characterized in that** the use encompasses tissue and/or organ protection in the case of or as a result of cerebral and/or myocardial ischemia, preferably in the case of impending reperfusion damage.

3. Composition for the use according to Claim 2, **characterized in that** the cerebral ischemia and the myocardial ischemia correlate with the clinical symptoms of stroke and of myocardial infarction.

4. Composition for the use according to Claim 1, **characterized in that** the use encompasses the prophylaxis and/or treatment of an indication selected from the group consisting of
- inflammatory states or inflammatory diseases,
- diseases associated with inflammatory tissue changes,
- rejections following a transplant and
- autoimmune diseases.

5. Composition for the use according to Claim 4, **characterized in that** the inflammatory states or inflammatory diseases are selected from the group consisting of rheumatoid arthritis, chronic polyarthritis, juvenile idiopathic arthritis, spondylitis, osteoarthritis, sepsis, septic shock, cerebral malaria, a chronic inflammatory lung disease, silicosis, sarcoidosis, reperfusion syndrome, inflammatory neurodegenerative diseases, including Crohn's disease, multiple sclerosis and Parkinson's disease, ulcerative colitis, depressions and fever in the case of infections.

6. Composition for the use according to Claim 4, **characterized in that** the diseases associated with inflammatory tissue changes are selected from the group consisting of Alzheimer's disease, Parkinson's disease and cancer.

7. Composition for the use according to any of the preceding claims, **characterized in that** the sulfoalkyl ether β-cyclodextrin is a sulfobutyl ether β-cyclodextrin or a sulfoethyl ether β Cyclodextrin.

8. Composition for the use according to any of the preceding claims, **characterized in that** the degree of substitution of the cyclodextrin with sulfoalkyl ether groups is from 3 to 8, preferably from 4 to 8, more preferably from 5 to 8, more preferably from 6 to 7.

9. Composition for the use according to any of the preceding claims, **characterized in that** the composition comprises a therapeutically effective amount of the complex composed of delphinidin and sulfoalkyl ether β Cyclodextrin.

10. Composition for the use according to any of the preceding claims, **characterized in that** the composition is used as a monopreparation.

11. Composition for the use according to any of Claims 1-9, **characterized in that** the composition comprises at least one further therapeutically active substance.

12. Composition for the use according to claim 11, **characterized in that** the at least one further therapeutically active substance is selected from the group consisting of the group of the anti-inflammatories, of the antibodies against inflammatory cytokines, of the soluble receptors of inflammatory cytokines or of the immunosuppressants and is preferably selected from the group consisting of acetylsalicylic acid, diclofenac, indomethacin, cyclosporine, azathioprine, bortezomib, melphalan, prednisone, vincristine, carmustine, cyclophosphamide, dexamethasone, thalidomide, doxorubicin, cisplatin, etoposide and cytarabine.

13. Composition for the use according to any of the preceding claims, further comprising one or more pharmaceutical excipients and/or additives, preferably selected from the group consisting of a pharmaceutically acceptable carrier, fillers, odorants and stabilizers.

14. Composition for the use according to any of the preceding claims in a formulation form for administration in a form selected from the group consisting of oral, rectal, parenteral, including intraperitoneal, percutaneous, subcutaneous, intramuscular, intravenous, ophthalmic, pulmonal and nasal.

15. Composition for the use according to Claim 14, **characterized in that** the administration form is selected from the group consisting of tablet, capsule, suspension, aerosol, solution, cream, paste, lotion, gel and ointment.

16. Composition for the use according to any of the preceding claims, **characterized in that** the complex composed of delphinidin and the sulfoalkyl ether β-cyclodextrin is used in a pharmaceutical preparation for the controlled and/or delayed release of the delphinidin.

## Revendications

1. Composition comprenant un complexe de delphinidine et d'une sulfoalkyléther-β-cyclodextrine, destinée à l'utilisation en tant qu'anti-inflammatoire et/ou immunosuppresseur.

2. Composition destinée à l'utilisation selon la revendication 1, **caractérisée en ce que** l'utilisation comprend la protection de tissus et/ou d'organes lors ou à la suite d'ischémie cérébrale et/ou d'ischémie myocardique, de préférence en cas de lésions imminentes de reperfusion.

3. Composition destinée à l'utilisation selon la revendication 2, **caractérisée en ce que** l'ischémie cérébrale et l'ischémie myocardique sont en corrélation avec les tableaux cliniques de l'accident vasculaire cérébral et de l'infarctus du myocarde.

4. Composition destinée à l'utilisation selon la revendication 1, **caractérisée en ce que** l'utilisation comprend la prophylaxie et/ou le traitement d'une indication choisie dans le groupe constitué par
- des états inflammatoires ou des maladies inflammatoires,
- des maladies associées à des altérations tissulaires inflammatoires,
- des réactions de rejet après une transplantation et
- des maladies auto-immunes.

5. Composition destinée à l'utilisation selon la revendication 4, **caractérisée en ce que** les états inflammatoires ou les maladies inflammatoires sont choisies dans le groupe constitué par la polyarthrite rhumatoïde, la polyarthrite chronique, l'arthrite idiopathique juvénile, la spondylite, l'ostéoarthrite, le sepsis, le choc septique, le paludisme cérébral, une maladie pulmonaire inflammatoire chronique, la silicose, la sarcoïdose, le syndrome de reperfusion, des maladies neurodégénératives inflammatoires, incluant la maladie de Crohn, la sclérose en plaques et la maladie de Parkinson, la colite ulcéreuses, des dépressions et la fièvre lors d'infections.

6. Composition destinée à l'utilisation selon la revendication 4, **caractérisée en ce que** les maladies associées à des altérations tissulaires inflammatoires sont choisies dans le groupe constitué par la maladie d'Alzheimer, la maladie de Parkinson et le cancer.

7. Composition destinée à l'utilisation selon l'une quelconque des revendications précédentes, **caractérisée en ce que** la sulfoalkyléther-β-cyclodextrine est une sulfobutyléther-β-cyclodextrine ou une sulfoéthyléther-β-cyclodextrine.

8. Composition destinée à l'utilisation selon l'une quelconque des revendications précédentes, **caractérisée en ce que** le degré de substitution de la cyclodextrine par des groupes sulfoalkyléther vaut de 3 à 8, de préférence de 4 à 8, de façon plus particulièrement préférée de 5 à 8, de façon plus particulièrement préférée de 6 à 7.

9. Composition destinée à l'utilisation selon l'une quelconque des revendications précédentes, **caractérisée en ce que** la composition comprend une quantité thérapeutiquement efficace du complexe de delphinidine et sulfoalkyléther-β-cyclodextrine.

10. Composition destinée à l'utilisation selon l'une quelconque des revendications précédentes, **caractérisée en ce que** la composition est utilisée sous forme de mono-préparation.

11. Composition destinée à l'utilisation selon l'une quelconque des revendications 1 à 9, **caractérisée en ce que** la composition comprend au moins une autre substance thérapeutiquement active.

12. Composition destinée à l'utilisation selon la revendication 11, **caractérisée en ce que** ladite au moins une autre substance thérapeutiquement active est choisie dans le groupe consistant en le groupe des anti-inflammatoires, des anticorps contre des cytokines inflammatoires, des récepteurs solubles de cytokines inflammatoires ou des immunosuppresseurs et est choisie de préférence dans le groupe constitué par l'acide acétylsalicylique, le diclophénac, l'indométacine, la ciclosporine, l'azathioprine, le bortézomib, le melphalan, la prednisone, la vincristine, la carmustine, le cyclophosphamide, la dexaméthasone, le thalidomide, la doxorubicine, le cisplatine, l'étoposide et la cytarabine.

13. Composition destinée à l'utilisation selon l'une quelconque des revendications précédentes, comprenant encore un ou plusieurs adjuvants et/ou additifs pharmaceutiques, de préférence choisis dans le groupe constitué par un véhicule pharmaceutiquement acceptable, des charges, des parfums et des stabilisants.

14. Composition destinée à l'utilisation selon l'une quelconque des revendications précédentes, sous une forme de formulation destinée à l'administration sous une forme choisie dans le groupe constitué par les formes orale, rectale, parentérale, y compris intrapéritonéale, percutanée, sous-cutanée, intramusculaire, intraveineuse, ophtalmique, pulmonaire et nasale.

15. Composition destinée à l'utilisation selon la revendication 14, **caractérisée en ce que** la forme d'administration est choisie dans le groupe constitué par des comprimés, des gélules, une suspension, une composition à pulvériser en aérosol, une solution, une crème, une pâte, une lotion, un gel et une pommade.

16. Composition destinée à l'utilisation selon l'une quelconque des revendications précédentes, **caractérisée en ce que** le complexe de delphinidine et de la sulfoalkyléther-β-cyclodextrine est utilisée dans une préparation galénique destinée à la libération programmée et/ou retardée de la delphinidine.
